(19) Europäisches Patentamt
European Patent Office
Office européen des brevets

(11) Publication number: **0 403 090 A1**

# EUROPEAN PATENT APPLICATION

(21) Application number: **90305552.3**

(51) Int. Cl.[5]: **C07D 493/08, A61K 31/415**

(22) Date of filing: **22.05.90**

(30) Priority: **12.06.89 US 364408**

(43) Date of publication of application: **19.12.90 Bulletin 90/51**

(84) Designated Contracting States: **AT BE CH DE DK ES FR GB GR IT LI LU NL SE**

(71) Applicant: **E.R. SQUIBB & SONS, INC.**
**Lawrenceville-Princeton Road**
**Princeton New Jersey 08543-400(US)**

(72) Inventor: **Stein, Philip David**
**64 Carter Road**
**Princeton, New Jersey(US)**
Inventor: **Hall, Steven Edward**
**57 Colleen Circle**
**Trenton, New Jersey(US)**

(74) Representative: **Thomas, Roger Tamlyn et al**
**D. Young & Co. 10 Staple Inn**
**London WC1V 7RD(GB)**

(54) **7-oxabicycloheptane imidazole prostaglandin analogs useful in the treatment of thrombotic and vasopastic disease.**

(57) 7-Oxabicycloheptane imidazole prostaglandin analogs are provided which are useful in treating thrombotic and vasospastic disease and have the structural formula

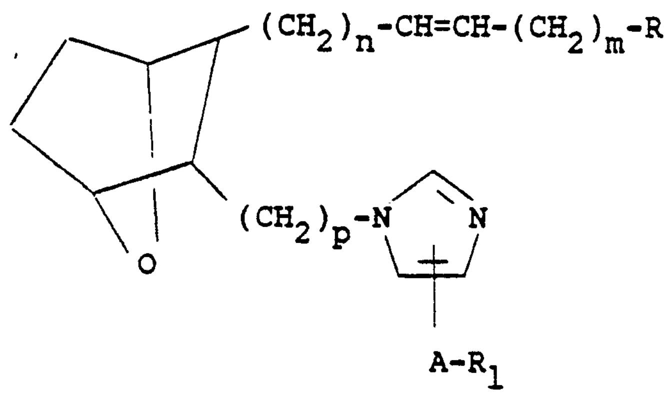

wherein m is 1, 2 or 3; n is 1, 2 or 3 and p is 1, 2 or 3; R is $CO_2H$, $CO_2$lower alkyl, $CO_2$alkali metal, $CONHSO_2R_2$ (wherein $R_2$ is lower alkyl or aryl) or 5-tetrazolyl; A is CHOH, C=O,

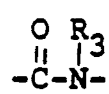

(wherein $R_3$ is H or lower alkyl), or a single bond; $R_1$ is lower alkyl, aryl, cycloalkyl or H, $R_1$ can be H only when A is a single bond.

EP 0 403 090 A1

# 7-OXABICYCLOHEPTANE IMIDAZOLE PROSTAGLANDIN ANALOGS USEFUL IN THE TREATMENT OF THROMBOTIC AND VASOSPASTIC DISEASE

The present invention relates to 7-oxabicycloheptane imidazole prostaglandin analogs which are cardiovascular agents useful, for example, in the treatment of thrombotic and/or vasospastic disease. These compounds have the structural formula

I

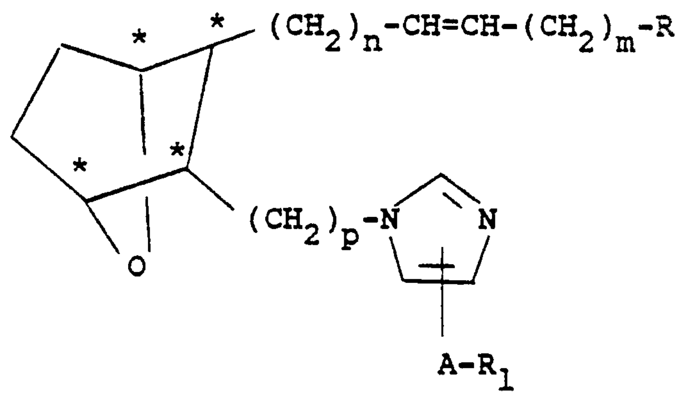

and including all stereoisomers thereof, wherein
m is 1, 2 or 3; n is 1, 2, or 3; and p is 1, 2 or 3;
R is $CO_2H$, $CO_2$lower alkyl, $CO_2$alkali metal $CONHSO_2R_2$ (wherein $R_2$ is lower alkyl or aryl) or 5-tetrazolyl;
A is CHOH,

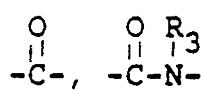

(wherein $R_3$ is H or lower alkyl) or a single bond; and
$R_1$ is H, lower alkyl, aryl, or cycloalkyl;
$R_1$ may be H only when A is a single bond.

Thus, the compounds of the invention encompass the following types of compounds:

IA

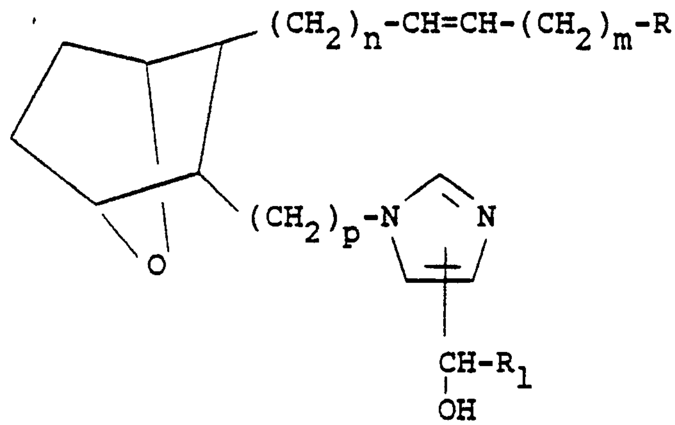

,

IB

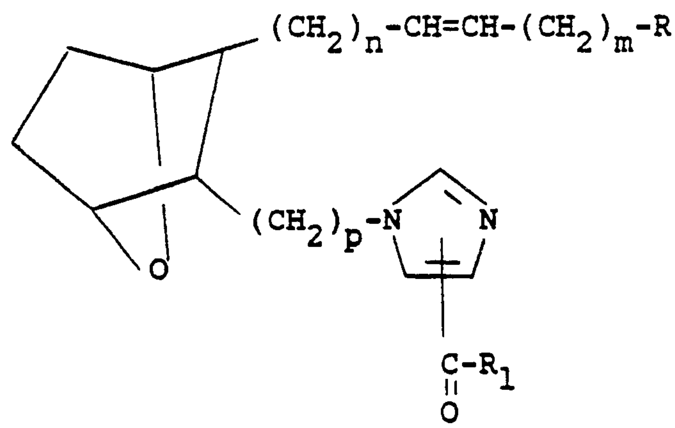

,

IC

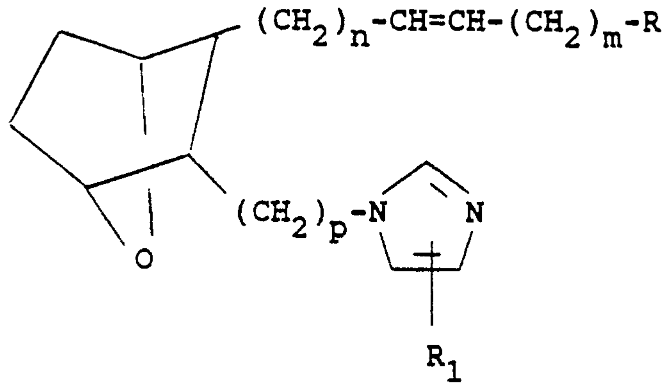

, and

ID

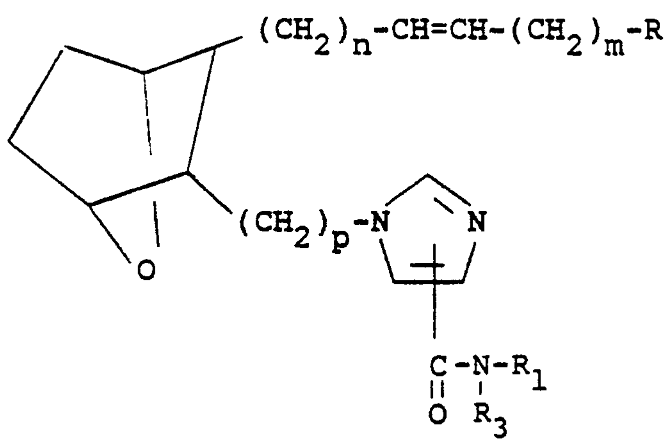

,

The term "lower alkyl" or "alkyl" as employed herein includes both straight and branched chain radicals of up to 12 carbons, preferably 1 to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including 1, 2 or 3 halo substituents, an aryl substituent, an alkyl-aryl substituent, a haloaryl substituent, a cycloalkyl substituent or an alkylcycloalkyl substituent.

The term "cycloalkyl" includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with 1 or 2 halogens, 1 or 2 lower alkyl groups and/or 1 or 2 lower alkoxy groups.

The term "aryl" or "Ar" as employed herein refers to monocyclic or bicyclic aromatic groups containing from 6 to 10 carbons in the ring portion, such as phenyl, naphthyl. Aryl (or Ar), phenyl or naphthyl may include substituted aryl, substituted phenyl or substituted naphthyl, which may include 1 or 2 substituents on either the phenyl or naphthyl such as lower alkyl, trifluoromethyl, halogen (Cl, Br or F), lower alkoxy, alkylthio, alkylsulfinyl, and/or alkylsulfonyl.

The term "aralkyl", "aryl-alkyl" or "aryl-lower alkyl" as used herein refers to lower alkyl groups as discussed above having an aryl substituent, such as benzyl.

The term "lower alkoxy", "alkoxy" or "aralkoxy" includes any of the above lower alkyl, alkyl or aralkyl groups linked to an oxygen atom.

The term "halogen" or "halo" as used herein refers to chlorine, bromine, fluorine or iodine with chlorine being preferred.

Preferred are those compounds of formula I wherein m is 2, n is 1, p is 1, R is $CO_2H$, A is CHOH, C = O, a single bond,

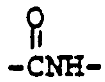

and $R_1$ is cycloalkylalkyl, or H, and -A-$R_1$ is in the 4-position of the imidazole ring.

The various compounds of the invention may be prepared as outlined below and as set out in the claims.

The compounds of formula I of the invention may be prepared as follows.

Compounds of the invention where A is CHOH are prepared starting with imidazole alcohol hydrochloride A

A 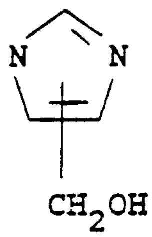 ·HCl

which is neutralized by passing A through a column of anion exchange resin, hydroxy form, to form the free base B

B 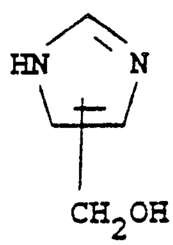

The free base B is then oxidized by treating a solution of B in an inert organic solvent such as dioxane, benzene or methylene chloride with manganese dioxide, or barium manganate under an inert atmosphere such as argon, to form the aldehyde C

C 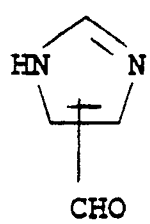

Aldehyde C is then made to undergo a Grignard reaction by teating C with a Grignard reagent of the structure

D MgBr-$R_1$

(prepared by adding bromide E

E Br-$R_1$

in an appropriate inert organic solvent such as tetrahydrofuran or diethyl ether to a stirred suspension of Mg turnings in an inert organic solvent such as tetrahydrofuran, or diethyl ether), employing a molar ratio of E : C of within the range of from about 1.5:1 to about 5:1, to form the alcohol F

F 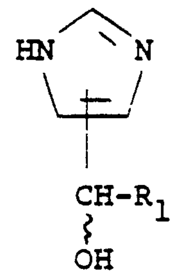

The alcohol F is treated with a protecting compound such as chlorodimethyl t-butylsilane in the presence of an amine base such as triethylamine and an inert organic solvent such as dimethyl formamide or methylene chloride, employing conventional procedures, to form the protected compound G

G 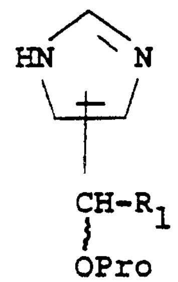

wherein Pro represents a protecting group.

Examples of protecting compounds suitable for use herein in reacting with alcohol F include but are not limited to

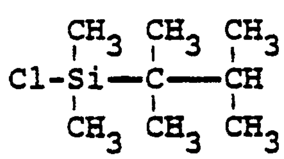

(chlorodimethyl-thexylsilane) ,

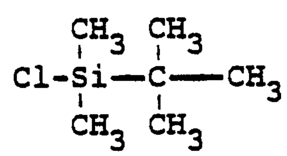

(chloro-dimethyl t-butylsilane) or

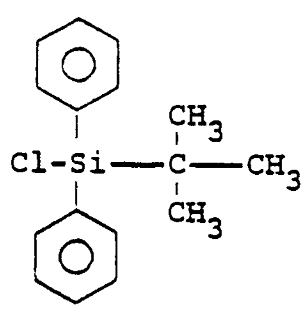

(t-butylchlorodiphenylsilane)

A solution of the protected alcohol G is formed in an inert solvent such as dimethylsulfoxide which is treated with a base such as sodium hydride (optionally in the presence of an inert carrier such as mineral oil) or lithium diisopropylamide (in tetrahydrofuran or hexanes). The resulting solution is treated with tosylate H

H

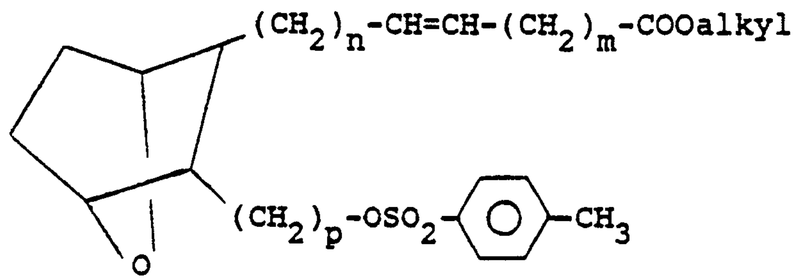

(prepared as described in U.S. Patent No. 4,663,336) employing a molar ratio of H : G of within the range of from about 1:1 to about 0.2:1 to form imidazole compound II

II

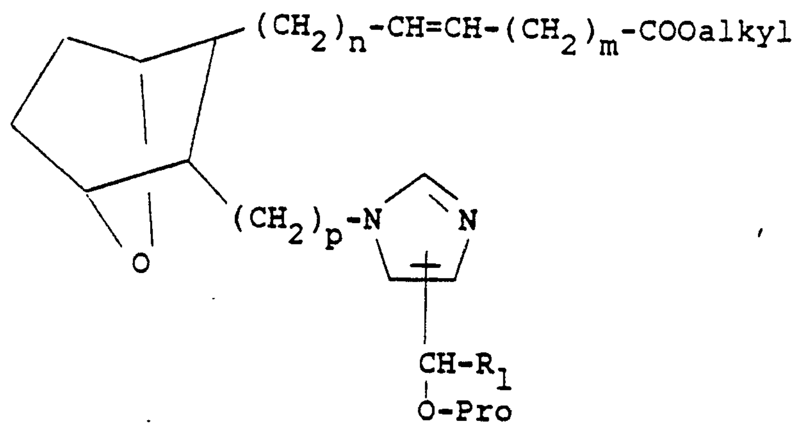

,

Compound II is then deprotected using standard procedures, for example by treating a solution of II in an alcohol solvent, such as methanol, under an inert atmosphere, such as argon, with acetyl chloride to form the corresponding alcohol IE

IE

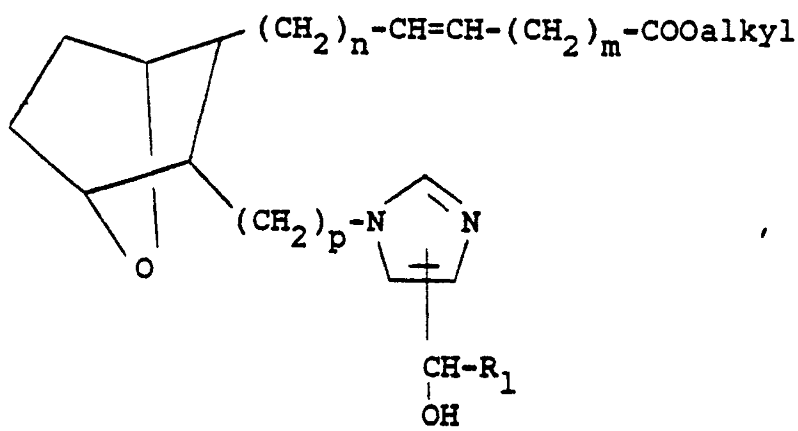

,

Compounds of the invention wherein A is C=O may be prepared by subjecting alcohol ester IE to allylic oxidation by treating a solution of IE in dioxane or benzene with an oxidizing agent such as activated manganese dioxide under an inert atmosphere such as argon, at a temperature within the range of from about 20°C to about 125°C, to form ester of the invention IF

IF

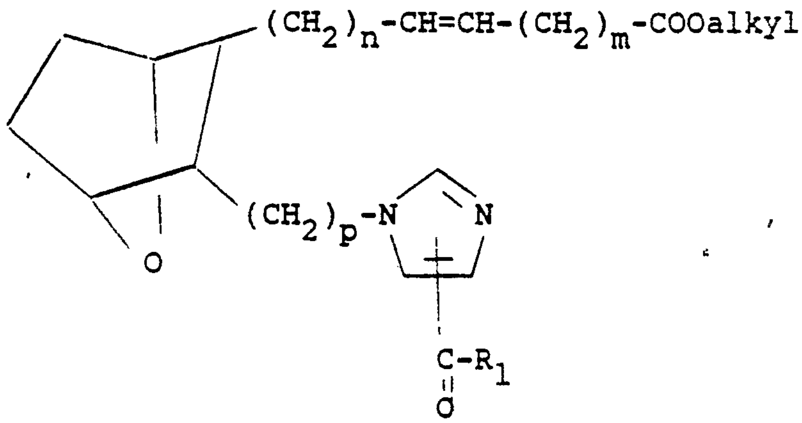

,

Compounds of the invention wherein A is a single bond and $R_1$ is other than H may be prepared by heating a solution of imidazole J

J

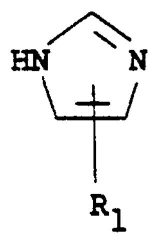

and tosylate H in anhydrous inert organic solvent such as anhydrous dimethylformamide, dimethyl sulfoxide or hexamethyl phosphoric triamide (HMPA), at a temperature within the range of from about 20 °C to about 125 °C, employing a molar ratio of J : H of within the range of from about 1:1 to about 5:1, to form ester of the invention IG

IG

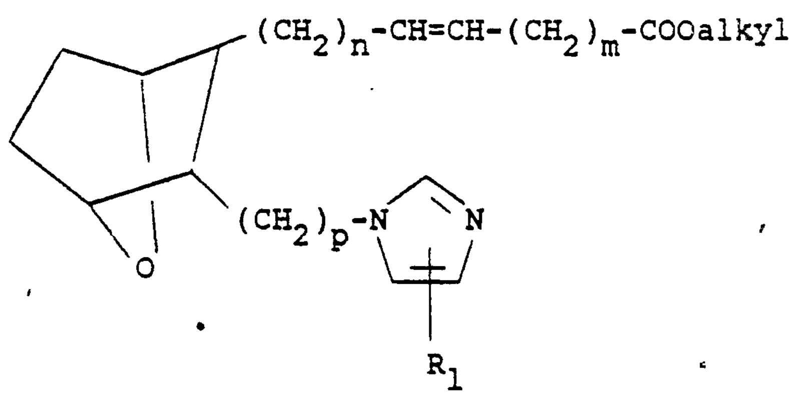

,

Compounds of the invention wherein A is a single bond and $R_1$ is H may be prepared by heating a solution of tosylate H and imidazole

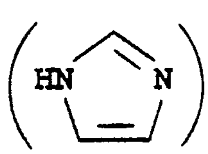

in an

inert organic solvent such as dimethylformamide, dimethyl sulfoxide or HMPA, under an inert atmosphere such as argon, at a temperature within the range of from about 20° C to about 125° C, employing a molar ratio of H:imidazole of within the range of from about 1.1:1 to about 0.17:1, to form the ester of the invention IH

IH

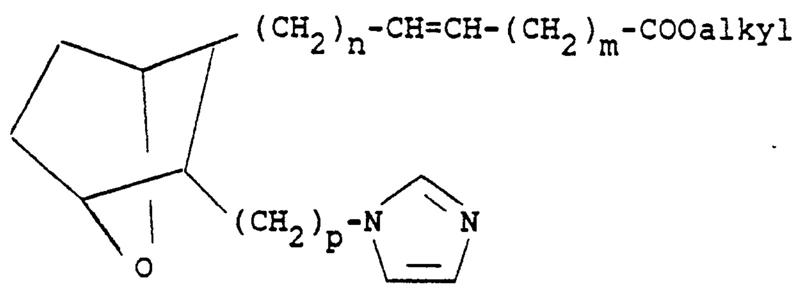

Compounds of the invention wherein A is

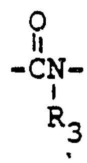

may be prepared by starting with imidazole carboxylic acid K

K

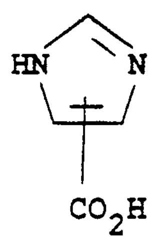

which is treated first with a carboxyl activating agent such as 1,1′-carbonyl diimidazole in an inert organic solvent such as dimethylformamide and subsequently with an amine salt of the formula L

L

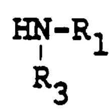

in the presence of a tertiary amine base such as triethylamine to form the amide M

M

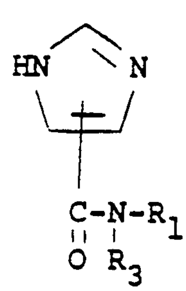

Amide M is then condensed with tosylate H, employing a molar ratio of H:M of within the range of from

about 1:1 to about 0.2:1, in the presence of base such as NaH, lithium diisopropyl amide or potassium t-butoxide, and an inert solvent such as dimethyl sulfoxide, dimethyl formamide or HMPA, at a temperature within the range of from about 20° C to about 125° C, to form compound IJ

IJ

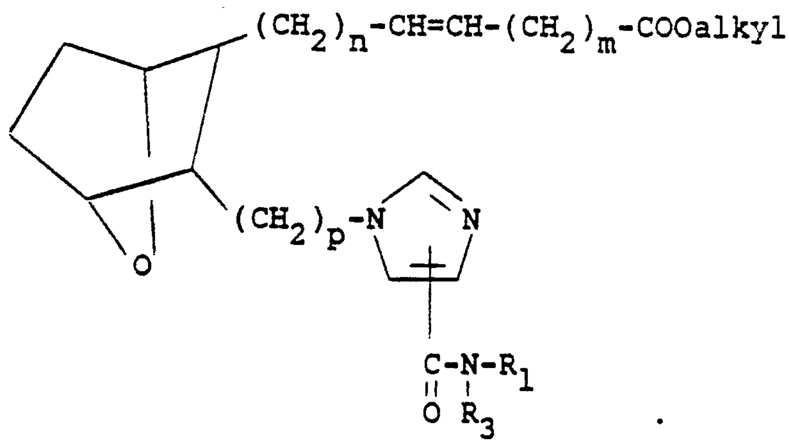

The esters IE, IF, IG, IH and IJ may be converted to the corresponding acids, that is IK

IK

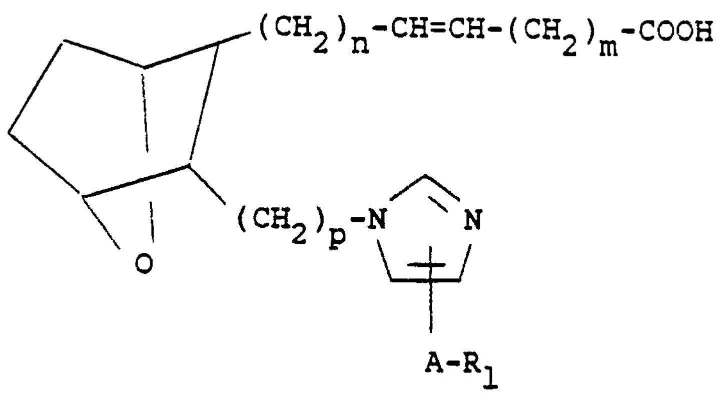

,

by treating the esters with a base, such as lithium hydroxide, sodium hydroxide or potassium hydroxide to form the corresponding alkali metal salt, followed by neutralization with an acid, such as dilute hydrochloric acid or oxalic acid to form the acid compounds of the invention.

Compounds of the invention wherein R is $CONHSO_2R_2$, that is IL

IL

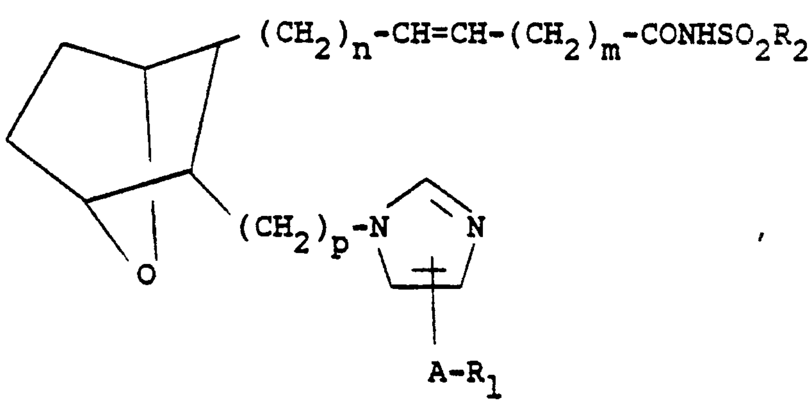

,

are prepared by treating acid IK with a sulfonamide of the structure P

P

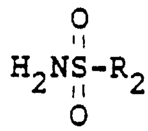

in the presence of a coupling agent such as carbonyldiimidazole or ethyl -3(3-dimethylamino)-propylcarbodiimide (WSC) and 4-dimethylaminopyridine, under an inert atmosphere such as argon, employing a molar ratio of P:IK of within the range of from about 0.8:1 to about 1.2:1, to form sulfonamide IL.

Compounds of the invention wherein R is 5-tetrazolyl, that is IM

IM

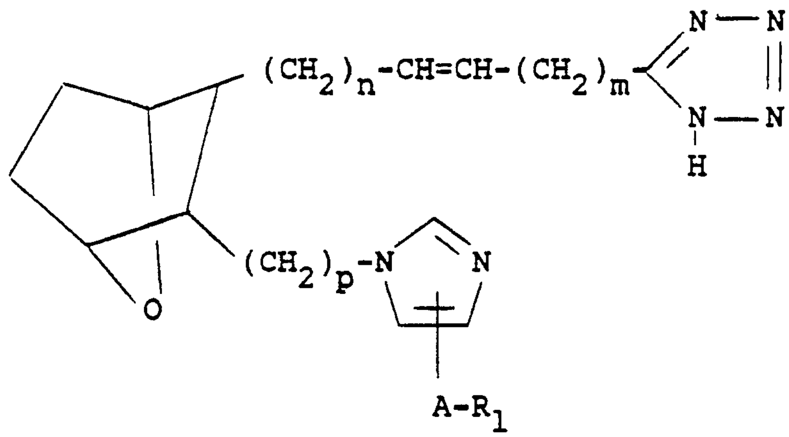

are prepared using the methodology set out above except substituting H′ for H to form protected tetrazole IN

H'

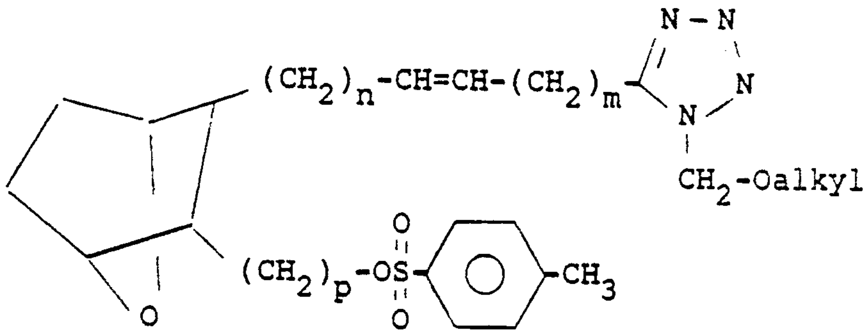

IN

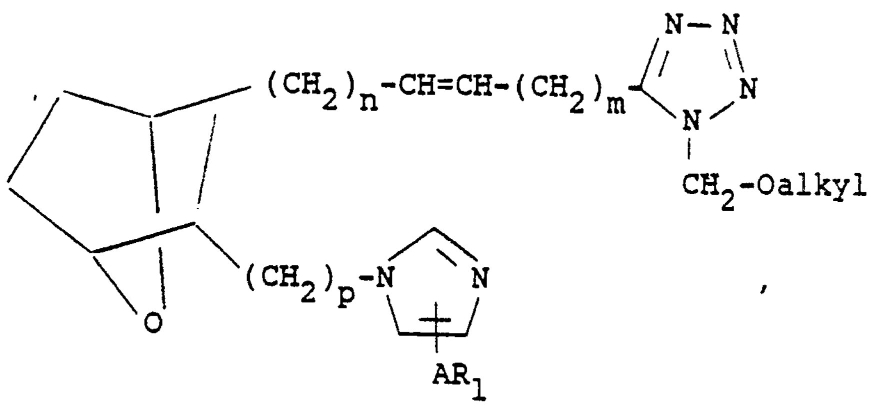

which is deprotected using methodology familiar to those skilled in the art to form IM. The starting tosylate H' is prepared from alcohol III (prepared as described in U.S. Patent 4,663,336)

III

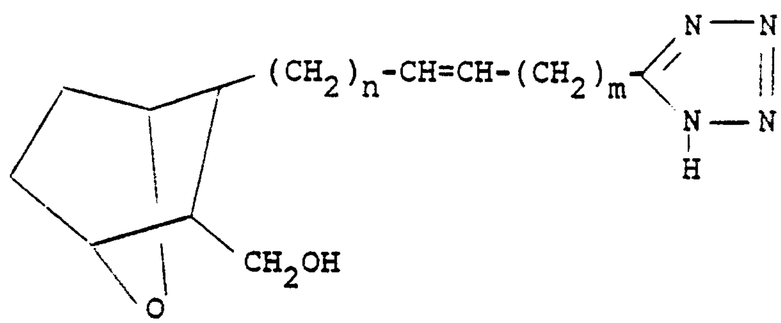

by treatment with a protecting agent such as Hal-$CH_2O$-alkyl in the presence of a base such as $K_2CO_3$ or NaH to form protected tetrazole IIIA

IIIA

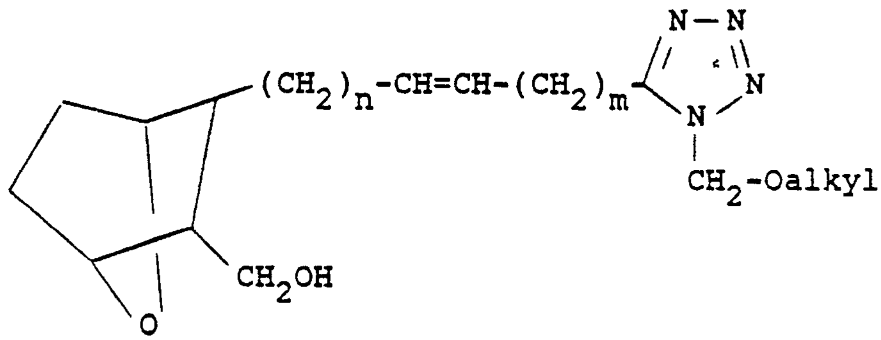

which is optionally homologated to alcohol IIIB

IIIB

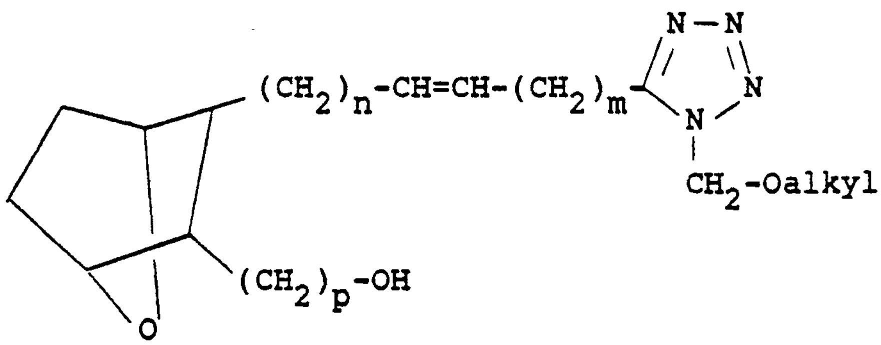

using the procedure as described in U.S. Patent 4,663,336. Alcohol IIIB is converted to the tosylate H′ by treatment with p-toluenesulfonyl chloride in an inert solvent such as methylene chloride and an amine such as pyridine.

The starting imidazole J in which A is attached at the 4 or 5 position of the imidazole ring may be prepared starting with acid Q

Q 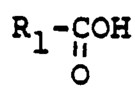

which is treated with oxalyl chloride to form the corresponding acid chloride R

R 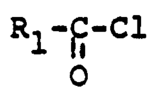

Acid chloride R is treated with cyanotrimethyl silane under an inert atmosphere such as argon, at a temperature within the range of from about 20° C to about 150° C, to form the nitrile S

S 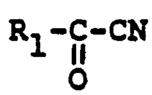

Nitrile S is made to undergo reductive acylation by treating S with a suspension of activated zinc dust in acetic anhydride and acetic acid, under an inert atmosphere such as argon, at a temperature within the range of from about 0° C to about 75° C, to form T

T 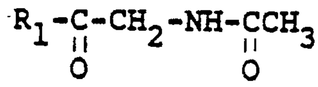

which is hydrolyzed by treating T with hydrochloric acid under an inert atmosphere such as argon to form the amine hydrochloride U

U 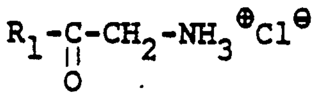

Compound U is then cyclized by reacting U with an aqueous solution of KSCN, at a temperature within the range of from about 20° C to about 100° C, employing a molar ratio of U:KSCN of within the range of from about 1:1 to about 0.25:1, to form imidazole thiol W

W 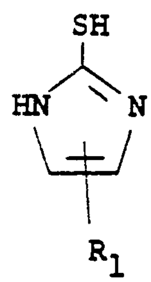

W is subjected to reductive desulfurization by treating W with a suspension of Raney nickel or other reducing agent in the presence of an inert organic solvent such as methanol or ethanol, under an inert atmosphere such as argon, at a temperature within the range of from about 20° C to about 90° C to form the starting imidazole J.

The starting imidazole J in which A is attached at the 2-position of the ring can be prepared by tretment of protected imidazole X with a strong base, for example, n-butyllithium, in an inert solvent such as THF to form the anion which is condensed with an

X 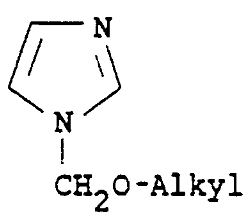

Y $Hal-R_1$

alkylating agent Y where Hal is a bromide, iodide or alkylsulfonate to form imidazole Z which is deprotected under standard conditions

Z 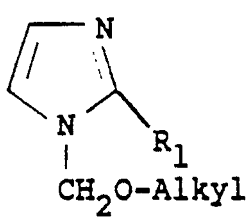

The compounds of this invention have four centers of asymmetry as indicated by the asterisks in

formula 1. However, it will be apparent that each of the formulae set out above which do not include asterisks still represent all of the possible stereoisomers thereof. All of the various stereoisomeric forms are within the scope of the invention.

The various stereoisomeric forms of the compounds of the invention, namely, cis-exo, cis-endo and all trans forms and stereoisomeric pairs may be prepared by employing starting materials and following the procedures as outlined in U.S. Patent No. 4,143,054. Examples of such stereoisomers are set out below.

Ia

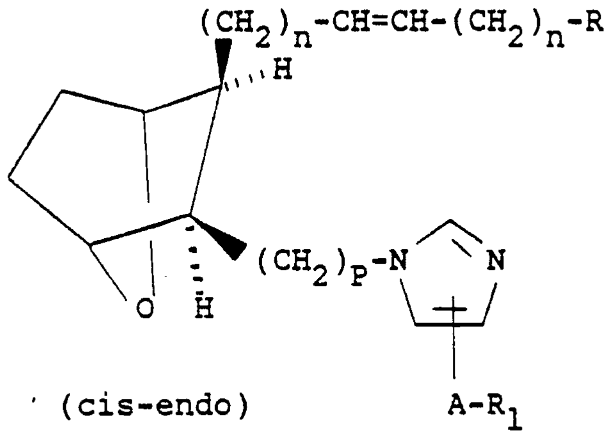

(cis-endo)

Ib

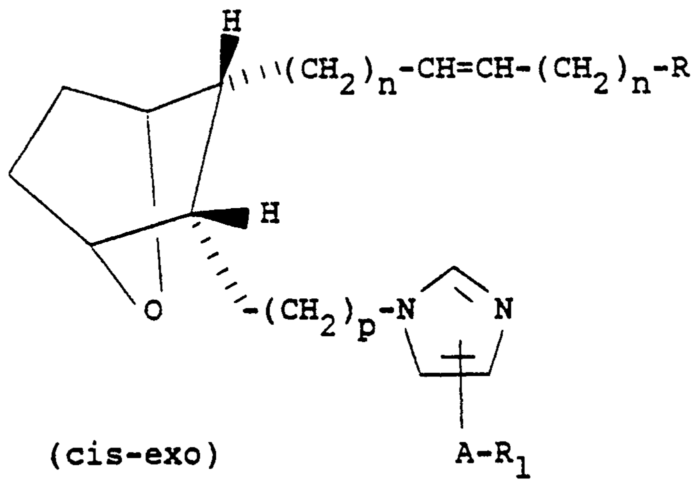

(cis-exo)

Ic

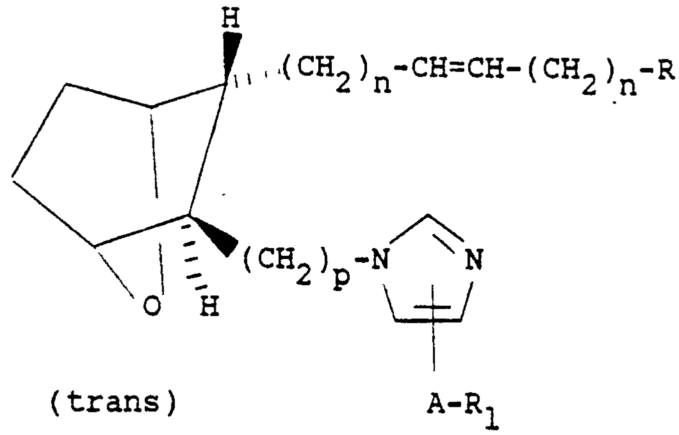

(trans)

Id

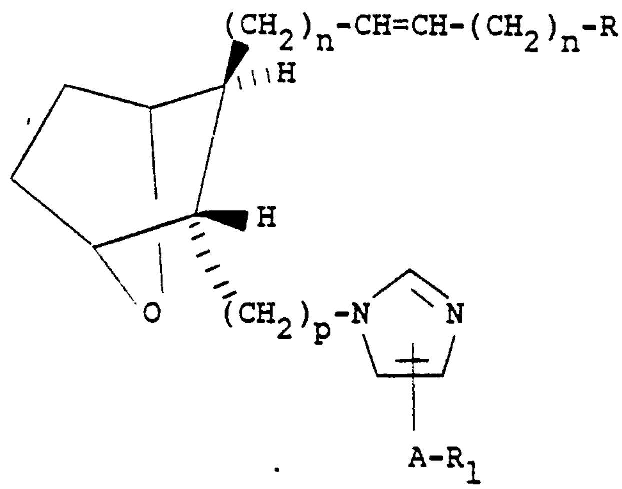

(trans)

The nucleus in each of the compounds of the invention is depicted as

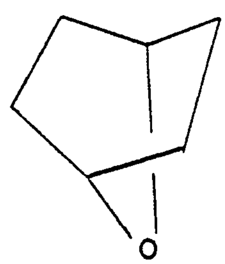

for matter of convenience; it will also be appreciated that the nucleus in the compounds of the invention may be depicted as

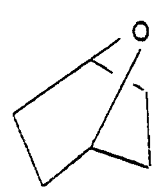

The compounds of this invention are thromboxane receptor antagonists and as such are useful as

inhibitors of thromboxane receptor mediated actions, and are also thromboxane synthetase inhibitors. The term "thromboxane receptor antagonist" includes compounds which are so-called thromboxane $A_2$ receptor antagonists, thromboxane $A_2$ antagonists, thromboxane $A_2$/prostaglandin endoperoxide antagonists, TP-receptor antagonists, or thromboxane antagonists.

The compounds of this invention are useful as inhibitors of platelet function, i.e., for the prevention and treatment of thrombotic vascular occlusive disorders, whether complete or partial, for example, arterial thrombosis, including that of the coronary, cerebral, ophthalmic, hepatic, mesenteric, renal, peripheral arteries or vascular or organ grafts, unstable angina, transient ischemic attacks, or intermittent claudication. They may be useful to prevent thrombosis following vascular injury produced in the course of diagnostic or therapeutic procedures such as endarterectomy or angiography. The compounds may be useful in the treatment or prevention of disorders characterized by platelet consumption and/or activation, including, platelet activation, dysfunction, and/or loss during extracorporeal circulation, the use of radiographic contrast agents, thrombotic thrombocytopenia purpura, disseminated intravascular coagulation, purpura fulminans, hemolytic transfusion reaction, hemolytic uremic syndrome, systemic lupus, cyclosporine-induced renal toxicity, pulmonary hypertension, side effects from dialysis, or abdominal aortic aneurism repair. The compounds may be used in the treatment of venous thrombosis or embolism, including pulmonary embolism, deep venous thrombosis, hepatic vein thrombosis, and renal vein thrombosis.

The compounds of this invention are useful as inhibitors of arterial or venous vasoconstriction. Accordingly, they may be useful to prevent vasoconstriction associated with unstable angina, chronic stable angina, and variant, or Prinzmetal's angina, Raynaud's syndrome, migraine headache, vasospasm of the coronary, cerebral, ophthalmic, hepatic, mesenteric, renal, peripheral arteries or vascular grafts, vascular injury such as that associated with surgery or trauma. Hypertension of pregnancy, the hepato-renal syndrome, and pulmonary hypertension are additional examples of vasoconstrictive disorders treatable by the compounds of this invention.

The compounds of this invention are useful as inhibitors of bronchoconstriction, i.e., airway hyper-responsiveness, allergic bronchospasm, asthma, and bronchoconstrictive responses to environmental, infectious, noxious or mechanical stimuli.

The compounds of this invention are useful as inhibitors of ischemic and reperfusion injury to various tissues, including, myocardium, skin, brain, bowel, or kidney, alone or in combination with other agents intended to restore blood flow. For example, these compounds may be useful for improving postischemic myocardial function and decreasing myocardial infarct size. Ischemia caused by reduced blood flow during diagnostic or therapeutic procedures may benefit by treatment with these compounds, for example, they reduce the myocardial stunning observed after bypass surgery. In addition, they may be useful for reducing the tissue injury caused by a stroke.

The compounds of this invention may be useful in the prevention or treatment of other conditions including burns, diabetic retinopathy, and tardive dyskinesia. The compounds may be useful in potentiating diuretic-induced diuresis.

In addition, the thromboxane receptor antagonists of the invention may be used with a thrombolytic agent such as t-PA, streptokinase, urokinase, prourokinase or anisoylated plasminogen-streptokinase activator complex (APSAC) within 6 hours of a myocardial infarction. In such case, the thrombolytic agent may be used in amounts conventionally employed, for example, as disclosed in the Physicians' Desk Reference for reducing post-ischemic myocardial injury.

The compounds of the invention can be administered orally or parenterally to various mammalian species known to be subject to such maladies, e.g., humans, cats, dogs and the like in an effective amount within the dosage range of about 0.1 to about 100 mg/kg, preferably about 0.1 to about 50 mg/kg and especially about 2 to 25 mg/kg (or from about 5 to about 2500 mg, preferably from about 10 to about 2000 mg) on a regimen in single or 2 to 4 divided daily doses.

The active substance can be utilized in a composition such as tablet, capsule, solution or suspension containing about 5 to about 500 mg per unit of dosage of a compound or mixture of compounds of formula I or in topical form for wound healing (0.01 to 5% by weight compound of formula I, 1 to 5 treatments per day). They may be compounded in conventional matter with a physiologically acceptable vehicle or carrier, excipient, binder, preservative, stabilizer, flavor, etc., or with a topical carrier such as Plastibase (mineral oil gelled with polyethylene) as called for by accepted pharmaceutical practice. Also as indicated in the discussion above, certain members additionally serve as intermediates for other members of the group.

The compounds of the invention may also be administered topically to treat peripheral vascular diseases and as such may be formulated as a cream or ointment.

The following Examples represent preferred embodiments of the present invention. Unless otherwise indicated, all tempertures are expressed in degrees Centigrade.

Example 1

[1S-[1α,2α(Z),3α,4α]]-6-[3-[[4-(4-Cyclohexyl-1-hydroxybutyl)-1H-imidazol-1-yl]methyl]-7-oxabicyclo[2.2.1]-hept-2-yl]-4-hexenoic acid, methyl ester

A. 1H-Imidazole-4-methanol

The free base of 4-(hydroxymethyl) imidazole was prepared by passing 10.00 g of the hydrochloride (74.31 mmol) through a column of 187 mL of analytical grade anion exchange resin (Bio-Rad AG 1-X8, 100-200 mesh, hydroxide form, 1.2 meg/mL of resin bed). Elution with water was carried out until the eluent was no longer basic and no more of the free base was visible by TLC (silica, 90/10 $CH_2CL_2$/$CH_3OH$). Eluent volume was ca. 650 mL. Water was removed in vacuo and azeotroped with toluene to yield 7.21 g of a white crystalline solid:
$^1H$ NMR ($CDCl_3$/$CD_3OD$): δ 7.53, s (1H); 7.35, s (1H); 6.93, s (2H); 3.76, br s.

B. 1H-Imidazole-4-carboxaldehyde

A solution of Part A imidazole (7.21 g, 74.3 mmol) in 360 mL of dioxane with 86.9 g of activated $MnO_2$ - (220.5 mmol) was refluxed under argon for 40 minutes. After cooling to room temperature the suspension was filtered through a pad of Celite and rinsed with several portions of hot dioxane (55° C); total rinse volume was 300 mL. Dioxane was removed in vacuo to yield 2.68 g of a white solid. The filter cake was rinsed with 300 mL each of boiling dioxane. Evaporation of the combined filtrates yielded an off-white solid which was combined with the above solid. The crude product was suspended in 50 mL of ethyl acetate, collected by filtration, rinsed with 25 mL of ethyl acetate then diethyl ether. Yield: 5.81 g of a white solid: mp 169-170° C dec.;
$^1H$ NMR ($CDCl_3$/$CD_3OD$): δ 9.80, s (1H); 7.81, s (1H); 7.79 s (1H); 3.75, br s (1H).

C. (3-Bromopropyl)cyclohexane

Title compound was prepared as described in Kamm, 0; Marvel, C.S. in Organic Synthesis 1921, 1, 1-13, procedure G;
$^1H$ NMR: δ 3.38, t, J=7Hz (2H); 0.83-1.91, m (15H).
$^{13}C$ NMR: δ 37.0, 35.9, 34.2, 33.2, 30.4, 26.6, 26.3.

D. α-(3-Cyclohexylpropyl)-1H-imidazole-4-methanol

To a stirred suspension of Mg turnings (4.40 g, 181.1 mmol) in 117 mL of tetrahydrofuran (THF) (room temperature, under argon) was added a few drops of a solution of 30.96 g (150.9 mmol) of Part C bromide in 25 mL of THF. Initiation was verified by the addition of a crystal of $I_2$ which immediately decolorized. The remaining solution of Part C bromide was then added dropwise so as to maintain a gentle reflux. After completion of the addition, stirring was continued for 45 minutes. Compound Part C bromide was completely consumed by TLC (silica, 75/25 hexanes/ethyl acetate two times, PMA). Part B compound (5.80 g, 60.4 mmol) was added in several increments and did not dissolve well. Reflux was initiated for 1 minute after which time most of Part B compound dissolved and the reaction proceeded vigorously without external heat application for 90 minutes. Reflux was subsequently carried out for 20 minutes. Complete consumption of part B compound was indicated by TLC. After cooling to room temperature, the reaction mixture was poured over 72 mL of 20% aqueous HCl solution (0° C) and stirred for 5 minutes. THF was removed in vacuo, 200 mL of water were added, two extractions with 40 mL each of $CHCl_3$ were performed, and the reaction mixture was brought to pH 8.0 with 2M aqueous NaOH solution. The crude product was extracted 3 times with ethyl acetate (450 mL total), dried over $Na_2SO_4$ and concentrated to yield 13.33 g of an off-white sticky solid (99%). Trituration from 200 mL of diethyl ether (2 crops) yielded 12.57 g of a white crystalline solid: mp decomp >88° C;

[1] NMR: δ 9.29, br s (2H); 7.71 br s (1H); 6.85, br s (1H); 4.71, br s (1H); 1.78, br s (2H); 1.66, br s and 1.62, br s (5H); 1.41-1.15, m (8H); 0.83, m (2H).
$^{13}C$ NMR: δ 139.66, 134.68, 115.76, 66.49, 37.55, 37.62, 37.03, 33.34, 26.66, 26.35, 23.09.

E. 4-[4-Cyclohexyl-1-[[(1,1-dimethylethyl)dimethylsilyl]oxy]butyl]-1H-imidazole

Triethyl amine (0.66 mL, 4.7 mmol) was added to a solution of Part D compound (1.0 g, 4.5 mmol) and 1.02 g (6.75 mmol) of t-butyldimethylsilyl chloride in 50 mL of $CH_2Cl_2$ (room temperature, under argon). The initially cloudy solution was homogeneous after 24 hours reaction time. Methylene chloride was removed in vacuo and replaced with ethyl acetate. The ethyl acetate solution was washed 3 times with saturated aqueous $K_2CO_3$ solution, brine, dried over $Na_2SO_4$, and concentrated to yield 1.48 g of a viscous light yellow oil (98%). Flash chromatography on 100 g of silica gel (E. Merck Kieselgel 60, 200-400 mesh, 98/2 $CH_2CL_2/CH_3OH$ yielded 1.31 g of a viscous colorless oil which very slowly crystallized to title compound in the form of a white solid upon standing:
$^1H$ NMR: δ 7.55, s (1H); 6.86, s (1H); 4.79, t (2H); 1.67-1.64, m (7H); 1.42-1.16, m (8H); 0.88, s and 0.88-0.80, m (11H); 0.06, s (3H); -0.06, s (3H).
$^{13}C$ NMR: δ 134.33, 118.18, 68.71, 39.25, 37.55, 37.29, 33.34, 26.72, 26.40, 25.83, 22.49, 18.17, -4.86, -4.96.

F. [1S-[1α,2α(Z),3α,4α]]-6-[3-[[4-[4-Cyclohexyl-1-[[(1,1-dimethylethyl)dimethylsilyl]oxy]butyl]-1H-imidazol-1-yl]methyl]-7-oxabicyclo[2.2.1]hept-2-yl]-4-hexenoic acid, methyl ester

A solution of 1.10 g of Part E compound (3.27 mmol) in 20 mL of dimethylsulfoxide (room temperature, under argon) was treated with 0.13 g of NaH (3.27 mmol of a 60% mineral oil dispersion). The NaH, added in several increments, was washed several times with pentane prior to the addition. Stirring at room temperature was continued for 30 minutes. The initally cloudy solution cleared up after 10 minutes. [1S-[1α,2α(Z),3α,4α]]-6-[3-[[[(4-Methylphenyl)sulfonyl]oxy]methyl]-7-oxabicyclo[2.2.1]hept-2-yl]-4-hexenoic acid, methyl ester prepared as described in Example 9, Part E, was added in 1 portion (1.21 g, 2.97 mmol) and the reaction was run at 90°C (oil bath temperature) for 4 hours. DMSO was removed with a vacuum pump/dry ice-cooled receiver flask. Ethyl acetate (50 mL) and 50 mL of water were added, the aqueous layer was extracted 2 times with 20 mL each of ethyl acetate, the combined ethyl acetate layers were washed with brine, dried over $Na_2SO_4$ and evaporated in vacuo to yield 1.76 g of a viscous yellow oil (>100%). Flash chromatography on 100 g of silica gel (E. Merck Kieselgel 60, 240-400 mesh) yielded 0.88 g of a viscous yellow oil:
$^1H$ NMR: δ 7.47, s; 7.40 (1H); 6.78, s (1H); 5.42-5.35 (3H); 4.72, t (1H); 4.27-4.09, m (2H); 3.95-3.88, m (1H); 3.79, t, J=11.14Hz (1H); 3.67, s (3H); 2.39, s (4H); 2.33-1.97, m (1H); 1.68-1.64, (m 8H); 1.49-1.16, m (7H); 0.95-0.83, m, 0.89, s, and 0.88, s (9H); 0.06, s, 0.00, s, -0.46, -0.06, s and 0.01 and -0.10, m (7H).

G. [1S-[1α,2α(Z),3α,4α]]-6-[3-[[4-(4-Cyclohexyl-1-hydroxybutyl)-1H-imidazol-1-yl]methyl]-7-oxabicyclo[2.2.1]-hept-2-yl]-4-hexenoic acid,methyl ester

A solution of Part F compound (0.59 g, 1.0 mmol) in 20 mL of methanol (room temperature, under argon) was treated with 0.50 mL of acetyl chloride (5.5 mmol). Stirring was continued for 1 hour. TLC (silica, 90/10 $CH_2CL_2/CH_3OH$ indicated an incomplete reaction. An additional portion of acetyl chloride was added and stirring continued for 90 minutes. Methanol was removed in vacuo. The residue was dissolved in 20 mL of ethyl acetate, washed 2 times with 30 mL each of saturated aqueous $KHCO_3$ solution, brine, dried over $Na_2SO_4$ and concentrated to yield 0.50 g of a viscous tan oil. Flash chromatography cn 35 g of silica gel (E. Merck Kieselgel 60, 240-400 mesh) yielded 0.29 g of a colorless oil. The product was eluted with 97/3 $CH_2Cl_2/CH_3OH$ after all upper $R_f$ by-products had been eluted with 99/1 $CH_2Cl_2/CH_3OH$. Yield: 0.61 g of title compound in the form of a colorless oil:
$^1H$ NMR: δ 7.45, s (1H); 6.83, s (1H); 5.43-5.34, m (2H); 4.64, br t (1H); 4.27, d, J=4.11Hz (1H); 4.15, d, J=4.11Hz (1H); 3.96, dd, J=4.69Hz, J=13.48Hz (1H); 3.81, t, J=13.48Hz (1H); 3.67, s (3H); 3.58, br m (1H); 2.39, s (4H); 2.29-2.00, m (4H); 1.97-1.76, m (2H); 1.70-1.65, m (7H); 1.49-1.37, m (4H); 1.23-1.19, m (6H); 0.86, m (2H).
$^{13}C$ NMR: δ 173.21, 146.28, 136.47, 129.38, 129.21, 114.49, 80.46, 78.44, 68.30, 51.40, 47.77, 46.13, 46.02, 37.41, 37.20, 37.09, 33.58, 33.23, 29.54, 28.85, 26.58, 26.26, 25.97, 22.89, 22.83.

Example 2

[1S-[1α,2α(Z),3α,4α]]-6-[3-[[4-(4-Cyclohexyl-1-hydroxybutyl)-1H-imidazol-1-yl]methyl]-7-oxabicyclo[2.2.1]-hept-2-yl]-4-hexenoic acid

A solution of 0.29 g of Example 1 compound (0.63 mmol) in 3.0 mL of $CH_3OH$ was treated with 0.35 g of KOH (6.3 mmol) then 2.5 mL of water (room temperature, under argon). The initially cloudy reaction mixture turned clear after 10 minutes. Stirring was continued for 1 hour and 20 minutes. Methanol was removed in vacuo. Water (2.0 mL) was added followed by 1M aqueous HCl solution to pH 1.0. Water was removed in vacuo. The residue was azeotroped with toluene. The residue was treated with 20 mL of 50/50 $CH_2Cl_2/CH_3OH$; insoluble salts were removed by filtration. The filtrate was concentrated and the above process repeated. The crude product after solvent removal contained some unreacted Example 1 compound. Hydrolysis of Example 1 compound was carried out again under the same conditions overnight. Workup procedure yielded 0.27 g of an off-white glass (96%), free of Example 1 compound by TLC. Flash chromatography on 20 g of silica gel (E. Merck Kieselgel 60, 240-400 mesh, 90/10 ethyl acetate-PAW * ) yielded 0.23 g of a viscous colorless oil. The oil was dissolved in 2.0 mL of $CHCl_3$ and filtered through a millipore filter (Gelman Acrodisc CR PTFE 0.45 µm), and evaporated in vacuo to yield an oil which was washed 3 times with 90/10 hexanes/ether (decanted off). Yield after solvent removal: 0.20 g of title compound as a white glassy solid;

$^1$H NMR: δ 9.10, br s (2H); 7.62, s (1H); 6.81, s (1H); 5.47-5.34, m (2H); 4.59, t (1H); 4.29, d (1H) and 4.10, d (1H), J=3.52Hz; 3.95, dd, J=13.5Hz, J=4.69Hz (1H); 3.82, t, J=13.50Hz (1H); 2.45-2.33, m and br d (4H); 2.26-1.93, m (4H); 1.80-1.65, m (9H); 1.49-1.43, m (1H); 1.35-1.09, m (9H); 0.93-0.83, m (2H).

$^{13}$C NMR: δ 171.36, 144.91, 136.27, 130.10, 129.15, 115.19, 80.86, 78.47, 67.30, 47.86, 46.71, 46.33, 37.58, 37.26, 36.63, 34.67, 33.38, 29.78, 28.84, 26.75, 26.44, 26.29, 23.41, 23.18.

| Anal. calc'd for $C_{25}H_{40}N_2O_4 \cdot 0.40H_2O \cdot 0.25HCl$: | |
|---|---|
| | C, 67.69; H, 8.98; N, 6.07; Cl, 1.92 |
| Found: | C, 67.85; H, 8.88; N, 5.91; Cl, 2.10. |

Example 3

[1S-[1α,2α(Z),3α,4α]]-6-[3-[[4-(3-Cyclohexylpropyl)1H-imidazol-1-yl]methyl]-7-oxabicyclo[2.2.1]hept-2-yl]-4-hexenoic acid, methyl ester

A. Cyclohexanebutanoyl chloride

To a solution of 15.0 g (0.088 mol) of cyclohexanebutyric acid in 30 mL of $CH_2Cl_2$ (0° C ice bath, under an argon stream) was added dropwise 8.45 mL (0.097 mol) of oxalyl chloride over 5 minutes. Vigorous gas evolution was observed shortly after completion of the oxalyl chloride addition. The reaction was run overnight at room temperature. An infrared scan of the reaction mixture indicated the presence of some unconverted acid. Dimethyl formamide (DMF) was added (20 drops) at room temperature (which caused immediate gas evolution) and the reaction was continued at room temperature for 45 minutes. Solvent was removed in vacuo to yield a light yellow oil.

*PAW = 20/6/11 pyridine/acetic acid/$H_2O$

B. α-Oxocyclohexanepentanenitrile

A solution of 47.1 mL (0.37 mol) of cyanotrimethyl silane in 58.44 g (0.31 mol) of Part A acid chloride was heated to 100° C (oil bath temperature) for 6 hours under argon. Reaction progress was monitored by infrared spectrometry ($CHCl_3$ solution). The reaction was carried out in a round-bottom flask fitted with a reflux condenser. Chlorotrimethylsilane was removed with a vacuum pump/dry ice-cooled receiver flask. Yield: 58.78 g of a red oil (title compound with some unremoved chlorotrimethylsilane):
$^{13}C$ NMR ($CDCl_3$, ref 77.0): δ 177.07, 113.26, 45.31, 37.24, 36.26, 33.07, 26.51 26.18, 20.23

C. N-(5-Cyclohexyl-2-oxopentyl)acetamide

To a stirred suspension of 50.28 g of activated zinc dust (0.77 mol) in 126 mL of acetic anhydride/126 mL of acetic acid under argon was added dropwise over 30 minutes a solution of 15.77 g (0.088 mol) of Part B compound in 17 mL of acetic anhydride/17 mL of acetic acid. The reaction temperature during and after the addition was maintained at 45° C (oil bath temperature). Reaction time was 4.5 hours. TLC (silica, 50/50 hexanes/ethylacetate) indicated complete conversion of Part B compound to product. The reaction mixture was cooled to room temperature, filtered through a pad of Celite, and rinsed with several portions of $CH_2Cl_2$. Removal of the solvents in vacuo (with several portions toluene as azeotrope) yielded 18.71 g of a yellow oil (94%). Flash chromatography on 750 g of E. Merck Kieselgel 60 silica gel (240-400 mesh) yielded 5.43 g of a white crystalline solid. The desired title product was eluted with 2L of 85/15 ethyl acetate-hexanes then ethyl acetate; upper-$R_f$ by-products were eluted with 50/50 hexanes/ethyl acetate then 2L of 75/25 ethyl acetate/hexanes: mp 76.5-78.5° C;
$^{13}C$ NMR ($CDCl_3$, ref 77.21): δ 205.91, 170.35, 49.55, 40.94, 39.78, 37.65, 37.15, 33.45, 26.86, 26.54, 23.17, 21.41

D. 1-Amino-5-cyclohexyl-2-propanone, monohydrochloride

A cloudy solution of Part C compound (14.97 g, 66.44 mmol) in 550 mL 4N HCl/139 mL tetrahydrofuran (THF) was refluxed under argon for 8.5 hours. The reaction mixture gradually became a clear yellow solution. THF and water were removed in vacuo (azeotroped with toluene). Trituration of the residue with 400 mL of diethyl ether yielded 13.87 g of title compound in the form of an off-white solid: mp 141-162° C dec.;
$^{13}C$ NMR ($CDCl_3/CD_3OD$, ref 77.00): δ 47.53, 40.49, 37.35, 36.75, 33.17, 26.51, 26.24, 20.39

E. 4-(3-Cyclohexylpropyl)-1H-imidazole-2-thiol

A solution of KSCN (1.93 g, 19.9 mmol) in 20 mL of water was treated with 3.36 g of Part D compound (15.3 mmol). The reaction. was run at 82° C (oil bath temperature) for 4.5 hours. Within minutes of heating, all of Part D compound dissolved to form a clear deep yellow solution. Product precipitation was first observed after c.a. 1 hour reaction time. The crude product was collected by filtration, rinsed several times with c.a. 25 mL total of water then 0° C chilled diethyl ether (50 mL total) to yield 2.66 g of title compound in the form of white crystalline plates: mp 176-177° C;
$^{13}C$ NMR ($CDCl_3$, ref 77.00): δ 157.56, 131.12, 111.13, 37.24, 36.64, 33.33, 26.56, 26.24, 25.53, 24.99

F. 4-(3-Cyclohexylpropyl)-1H-imidazole

A suspension of 11.0 g of W-2 Raney nickel in a somewhat cloudy solution of 2.30 g of Part E compound (10.25 mmol) in 100 mL of absolute methanol was refluxed under argon for 2.5 hours. After cooling to room temperature, the reaction mixture was filtered through a pad of Celite, rinsed with c.a. 30 mL each of absolute ethanol, methanol, then water. Evaporation of filtrate in vacuo (azeotroped 2 times with toluene then co-evaporated with diethyl ether) yielded 1.72 g of an off-white solid (87%). The crude product was partitioned between ethyl acetate and 25 mL of 2M aqueous trisodium citrate solution. After separation of the layers, the ethyl acetate layer was washed 2 times with 25 mL total of the above citrate solution (pH 8.5), brine, dried over $Na_2SO_4$ and evaporated in vacuo to yield 1.63 g of an oil which slowly crystallizes

upon standing. The solid was suspended in 30 mL of hexanes, collected by filtration and rinsed with c.a. 20 mL of hexanes. Yield: 1.60 g of title compound in the form of a white crystalline solid: mp 81-82° C; $^{13}C$ NMR ($CDCl_3$, ref 77.00): δ 136.78, 134.22, 117.86, 37.49, 37.12, 33.32, 26.89, 26.75, 26.66, 26.35

G. [1S-[1α,2α(Z),3α,4α]]-6-[3-[[4-(3-Cyclohexylpropyl)-1H-imidazol-1-yl]methyl]-7-oxabicyclo[2.2.1]hept-2-yl]-4-hexenoic acid, methyl ester

A solution of 0.71 g of Part F compound (3.7 mmol) and 0.50 g of [1S-[1α,2α(Z),3α,4α]]-6-[3-(tosyloxymethyl)-7-oxabicyclo[2.2.1]hept-2-yl]-4-hexenoic acid, methyl ester prepared as described in Example 9, Part E (1.2 mmol) in 2.0 mL of anhydrous DMF was stirred at 115° C (oil bath temperature) overnight under argon. DMF was removed with a vacuum pump-dry ice-cooled receiver flask. Ethyl acetate (30 mL) and water (15 mL) were added, the ethyl acetate layer was washed with water (15 mL), brine, dried over $Na_2SO_4$ and concentrated to yield 1.39 g of a dark tan viscous oil. Recrystallization from ethyl acetate (1 crop, seeded with Part F compound) yielded 0.25 g of an off-white crystalline solid, the tosylate salt of Part F compound. The filtrate (0.82 g of a yellow oil) was flash chromatographed on 30 g of E. Merck Kieselgel 60 silica gel (240-400 mesh, 99/1 $CH_2CL_2/CH_3OH$ then 95/5 $CH_2Cl_2/CH_3OH$ after most of the desired product has been eluted) to yield 0.25 g of title compound in the form of a light yellow viscous oil: $^{13}C$ NMR ($CDCl_3$, ref 77.00): δ 173.01, 143.49, 135.95, 129.35, 129.01, 114.46, 80.28, 78.33, 51.20, 47.66, 46.02, 45.70, 37.26, 36.94, 33.46, 33.11, 29.40, 28.77, 28.48, 26.40, 26.12, 25.86, 22.69

## Example 4

[1S-[1α,2α(Z),3α,4α]]-6-[3-[[4-(3-Cyclohexylpropyl)-1H-imidazol-1-yl]methyl]-7-oxabicyclo[2.2.1]hept-2-yl]-4-hexenoic acid

A solution of 0.25 g of Example 3 compound (0.58 mmol) in 3.0 mL of methanol was treated with 0.33 g (5.83 mmol) of KOH then 1.4 mL of water. Stirring of the clear yellow solution was continued under argon for 4.5 hours. 1M HCl was added to pH 1.0. Methanol and water were removed in vacuo. The crude product was dissolved in 40 mL of 1:1 methanol-methylene chloride and insoluble salts were removed by filtration. The filtrate was concentrated and the above process repeated. Removal of the solvents in vacuo yielded 0.28 g of a light yellow taffy. Flash chromatography on 28 g of E. Merck Kieselgel 60 silica gel (240-400 mesh, 95/5 ethyl acetate/PAW) followed by treatment of the residue 3 times with 3.0 mL each of 75/25 hexanes/diethyl ether and decanting yielded 0.13 g of title product in the form of a viscous yellow oil. The product was dissolved in 2.0 mL of ethyl acetate, filtered through a milipore filter, and evaporated. The above process was repeated once. Yield after solvent removal: 80.9 mg of hard yellow taffy:
$^{13}C$ NMR ($CDCl_3$, ref 77.00): δ 141.59, 136.18, 130.10, 129.12, 115.24, 80.78, 78.64, 47.89, 46.77, 46.42, 37.46, 37.06, 34.56, 33.38, 29.75, 28.80, 27.53, 26.72, 26.41, 26.32, 26.23, 23.35
$^1H$ NMR ($CDCl_3$, ref TMS): δ 7.81, s (1H); 6.64, s (1H), 5.47-5.40, m (2H); 4.29, d, J≅4.1Hz (1H); 4.13, d, J≅4.1Hz (1H); 3.96, dd, J=14.1Hz, J=4.69Hz (1H); 3.82, dd, J=14.1Hz, J=11.7Hz (1H); 2.54, t (2H); 2.38, m (4H); 2.23-1.97, m (3H); 1.67, m (8H); 1.48-1.37, m (4H); 1.20, m (5H); 0.88, m (2H).

| Anal. calc'd for $C_{25}H_{38}N_2O_3 \cdot 0.7HCl \cdot 0.5H_2O$: | |
|---|---|
| | C, 66.87; H, 8.91; N, 6.24; Cl, 5.53 |
| Found: | C, 66.90; H, 8.51; N, 5.99; Cl, 5.59. |

## Example 5

[1S-[1α,2α(Z),3α,4α]]-6-[3-[[4-(4-Cyclohexyl-1-oxobutyl)-1H-imidazol-1-yl]methyl]-7-oxabicyclo[2.2.1]hept-2-yl]-4-hexenoic acid, methyl ester

A solution of 0.50 g of Example 1 compound (1.09 mmol) with a suspension of 0.28 g of activated $MnO_2$ (3.0 eq.) in 15 mL of dioxane was refluxed under argon for 3 days. Reaction progress was slow, with repeated additions of $MnO_2$ necessary to drive the oxidation to completion (total amount of $MnO_2$ added was 13.5 eq., 0.14 g at a time). The reaction mixture was filtered through a pad of Celite and rinsed with boiling dioxane until no more product was visible by TLC in the filtrate (100 mL). Dioxane was removed in vacuo and replaced with 15 mL of ethyl acetate. The ethyl acetate solution was rinsed with 3 mL of 2M trisodium citrate solution, brine, dried over $Na_2SO_4$ and evaporated to yield 0.50 g of a viscous tan oil (>100%). The crude product was absorbed on Celite and filtered through a small pad of Florisil (2.4 x 2.2 cm); the pad was rinsed with hexanes and the desired product eluted with 95/5 ethyl acetate/$CH_3OH$. Yield: 0.41 g of title compound in the form of a yellow oil. NMR data indicated a small amount of impurity present:
$^1H$ NMR ($CDCl_3$, ref TMS): δ 7.54, d, J=1.76Hz (1H); 7.44, d, J=1.17Hz (1H); 5.35, m (2H); 4.21, d (1H) and 4.05, d, J=4.10Hz (1H); 3.93, dd, J=13.49Hz and J=4.69Hz (1H); 3.83, t, J=13.49Hz (1H); 3.60 s (3H); 2.88, t, J=11.14Hz (2H); 2.32, br s (4H); 2.24-1.71, m (4H); 1.66-1.58, m (9H); 1.44-1.30, m (2H); 1.22-1.10, m (6H); 0.82-0.78, m (2H)
$^{13}C$ NMR ($CDCl_3$, ref 77.00): δ 196.52, 172.95, 142.56, 137.29, 129.26, 129.09, 122.50, 80.35, 78.09, 51.25, 47.65, 46.39, 46.00, 38.75, 37.19, 36.85, 33.39, 33.00, 29.43, 28.67, 26.45, 26.11, 25.97, 22.71, 21.31.

## Example 6

[1S-[1α,2α(Z),3α,4α]]-6-[3-[[4-(4-Cyclohexyl-1-oxobutyl)-1H-imidazol-1-yl]methyl]-7-oxabicyclo[2.2.1]hept-2-yl]-4-hexenoic acid

A cloudy solution of 0.41 g of Example 5 compound (0.90 mmol) and 9.0 mL of 1M aqueous LiOH solution in 39 mL of THF/7.8 mL of water was stirred at room temperature for 2.5 hours. THF was removed in vacuo. The aqueous gum was extracted 6 times with 15 mL each of $CHCl_3$. The combined $CHCl_3$ layers were washed with brine, dried over $Na_2SO_4$ and evaporated in vacuo to yield 0.28 g of a light yellow taffy (70%). TLC indicated no product remained in the aqueous layer. Flash chromatography on 25 g of silica gel (E. Merck Kieselgel 60, 240-400 mesh, 99/1 ethyl acetate/PAW) yielded 0.28 g of title compound in the form of a viscous light yellow taffy. The product was washed 2 times with 5 mL each of hexanes at 0 °C; the hexanes were decanted off. Removal of residual hexanes yielded 0.25 g of title compound in the form of a light yellow viscous taffy.
$^1H$ NMR ($CDCl_3$, ref TMS): δ 10.78, br s (1H); 7.60, br s (1H); 7.56, br s (1H); 5.40-5.34, m (2H); 4.20, d (1H) and 4.10, d (1H), J=4.11Hz; 3.96, dd, J=11.14Hz and J=4.69Hz (1H); 3.86, t, J=11.14Hz (1H); 2.83, t, J=7.13Hz (2H); 2.34, br s (4H); 2.21-1.94, m (4H); 1.61-1.58, m (9H); 1.44-1.30, m (2H); 1.18-1.12, m (6H); 0.81, m (2H).
$^{13}C$ NMR ($CDCl_3$, ref 77.00): δ 196.42, 176.61, 141.96, 137.96, 129.64, 129.12, 122.96, 80.63, 78.32, 77.46, 47.80, 46.71, 46.16, 39.05, 37.38, 36.97, 33.69, 33.17, 29.54, 28.79, 26.58, 26.26, 26.12, 22.83, 21.42.

| Anal. calc'd for $C_{26}H_{38}N_2O_4 \cdot 0.75HCl$: | |
|---|---|
| | C, 66.44; H, 8.31; N, 5.96; Cl, 5.68 |
| Found: | C, 66.57; H, 8.14; N, 5.76; Cl, 5.76. |

## Example 7

[1S-[1α,2α(Z),3α,4α]]-6-[3-(1H-Imidazol-1-ylmethyl)-7-oxabicyclo[2.2.1]hept-2-yl]-4-hexenoic acid, methyl ester

A solution of 0.60 g of the tosylate employed in Example 3, Part G (1.47 mmol) with 0.30 g of imidazole (4.4 mmol) in 1.2 mL of dry DMF under argon was reacted at 110° C (oil bath temperature) overnight. Dimethylformamide (DMF) was removed with a vacuum pump/dry ice-cooled receiver flask. Ethyl acetate and aqueous 10% $KHCO_3$ solution were added, the ethyl acetate layer was washed 3 times with 10% aqueous $KHCO_3$ solution, 3 times with water, brine, dried over $Na_2SO_4$ and evaporated in vacuo to yield 0.37 g of a yellow oil (83%). Flash chromatography on 50 g of silica gel (E. Merck Kieselgel 60, 240-400 mesh) yielded 0.24 g of title product in the form of a viscous yellow oil. The product was eluted with 97/3 $CH_2Cl_2/CH_3OH$ after all upper-$R_f$ by-products were eluted with 99/1 $CH_2Cl_2/CH_3OH$:

$^{13}C$ NMR ($CDCl_3$, ref 77.00): δ 173.04, 136.96, 129.41, 129.26, 129.09, 118.61, 80.31, 78.27, 51.28, 47.74, 46.02, 45.81, 33.46, 29.40, 28.74, 25.86, 22.69

$^1H$ NMR ($CDCl_3$, ref TMS): δ 7.51, s (1H); 7.07, s (1H); 6.96, s (1H); 6.96-5.40, m (2H); 4.28, d, J=4.11Hz (1H); 4.14, d, J=4.11Hz (1H); 3.98, dd, J=11.14Hz, J=4.69Hz (1H); 3.87, t, J=11.14Hz, (1H); 3.67, s (3H); 2.39, br s (4H); 2.32-2.25, m (2H); 2.23-1.99, m (2H); 1.69, m (2H); 1.50-1.26, m (2H).

## Example 8

[1S-[1α,2α(Z),3α,4α]]-6-[3-(1H-Imidazol-1-ylmethyl)-7-oxabicyclo[2.2.1]hept-2-yl]-4-hexenoic acid

A solution of 0.15 g of Example 7 ester (0.49 mmol) and 0.28 g of KOH (4.9 mmol) in 1.2 mL of water/2.5 mL of methanol was stirred at room temperature under argon for 4.5 hours. Methanol was removed in vacuo and 1M HCl was added to pH 1.0. Water was removed in vacuo, azeotroped with toluene. The residue was suspended in 40 mL of 50/50 $CH_2CL_2/CH_3OH$ filtered through a pad of Celite, and rinsed with 2 x 20 mL portions 50/50 $CH_2CL_2/CH_3OH$. The filtrate was concentrated to yield 0.20 g of a yellow taffy which was suspended in hexanes 3 times (5 mL each, decanted off). Flash chromatography on 20 g of silica gel (E. Merck Kieselgel 60, 240-400 mesh, 5/1 ethyl acetate/PAW) yielded 69.0 mg of a hard tan taffy. The product was dissolved in 2.4 mL of ethyl acetate and filtered through a millipore filter. The above process was repeated on the filtrate. Yield after concentration of the filtrate: 30.0 mg of title compound in the form of a hard tan taffy which very slowly solidified to a white solid upon standing:

$^{13}C$ NMR ($CDCl_3$, ref 77.00): δ 130.16, 129.10, 80.80, 78.50, 48.06, 46.59, 46.39, 29.75, 28.97, 26.26, 23.35

$^1H$ NMR ($CDCl_3$, ref TMS): δ 12.41, br s (1H); 7.73, br s (1H); 7.12, br s (1H); 6.96, br s (1H); 5.48-5.41, m (2H); 4.30, br s (1H); 4.11, br s (1H); 3.95, br dd (1H); 3.88, br t (1H); 2.40, br s (4H); 2.24-1.99, m (4H); 1.68, br s (2H); 1.49-1.34, m (2H).

| Anal. calc'd for $C_{16}H_{22}N_2O_3 \cdot 0.06HCl \cdot 0.43H_2O$: | |
|---|---|
| | C, 63.98; H, 7.69; N, 9.33; Cl, 0.71 |
| Found: | C, 64.06; H, 7.73; N, 8.80; Cl, 0.78. |

## Example 9

[1S-[1α,2α(Z),3α,4α]]-6-[3-[[4-[[(4-Cyclohexylbutyl)amino]carbonyl]-1H-imidazol-1-yl]methyl]-7-oxabicyclo-[2.2.1]hept-2-yl]-4-hexenoic acid, methyl ester

All chromatographies were performed with E. Merck Kieselgel 60 silica gel (240-400 mesh).

A. 1H-Imidazole-4-carboxylic acid

Compound A was prepared according to the method of Cohen, David, & Kirk (J. Het. Chem., 19, 253

(1982)).

B. 4-Cyclohexylbutylamine hydrochloride

To a stirred solution of 4-phenylbutylamine (10.6 g, 71.1 mmol, Aldrich) in 100 mL of glacial acetic acid under argon was added 87% $PtO_2$ (1.06 g, 10% weight based on 4-phenylbutylamine). The reaction mixture was hydrogenated at 54 psi at room temperature for 4 hours. The catalyst was removed by filtration through a 2" pad of Celite, and the filtrate was concentrated in vacuo. The residue was diluted with 200 mL of diethyl ether, 100 mL of $CH_3OH$ and 8 mL of concentrated HCl. This mixture was concentrated in vacuo and triturated in diethyl ether to give 13.1 g (97%) of desired amine•HCl.

C. [1S-[1α,2α(Z),3α,4α]]-6-[3-(Hydroxymethyl)-7-oxabicyclo[2.2.1]hept-2-yl]-4-hexenoic acid, methyl ester

To a partial solution of 36.27 g of (endo)-octahydro-5,8-epoxy-1H-benzo[c]pyran-3-ol (prepared as described in U.S. Patent No. 4,143,054) (0.23 mol) and 3-carboxypropyltriphenylphosphonium bromide (127.34 g, 0.37 mol) in 600 mL of dry THF under argon at 3° C was added dropwise over 1 hour a solution of 370.6 mL of potassium t-amylate (0.68 mol of a 1.8M toluene solution) with mechanical stirring. Initially the reaction temperature reached a maximum of 8° C and subsequently leveled off to 4° C for the remainder of the base addition. The reaction was then run at room temperature for 90 minutes. A 0° C ice bath was introduced and the reaction was quenched by the addition of 152 mL of glacial acetic acid, over 30 minutes. Solvents were removed in vacuo (azeotroped with toluene). Water (640 mL) and 50 mL of concentrated HCl were added (pH 2.6). Dilution with 640 mL of ethyl acetate, the addition of 149 g of NaCl and a few seed crystals of 3-carboxypropyltriphenylphosphonium bromide was followed by vigorous stirring for 15 minutes. The precipitate was collected by filtration and washed with 2 portions each of 320 mL of ethyl acetate. The ethyl acetate layer was separated, the aqueous layer was extracted with ethyl acetate (2 x 200 mL each), the combined ethyl acetate layers were dried over $MgSO_4$ and concentrated. Aqueous 5% $K_2CO_3$ was added (507 mL) followed by vigorous stirring for 1 hour. No precipitation occurred. The reaction mixture was concentrated to a paste and suspended in 508 mL of water. Several hours of vigorous stirring produced no precipitate. The water was decanted off and the residue was suspended in 200 mL of aqueous 5% $K_2CO_3$ solution. After vigorous stirring, a light tan solid was collected by filtration and rinsed several times with water. The combined aqueous layers were extracted 5 times with 1:1 toluene/diethyl ether (230 mL each). After cooling the combined aqueous layers with a 0° C ice bath, concentrated HCl was added to pH 2.5, followed by extraction once with 460 mL then 2 times with 230 mL each of ethyl acetate. The combined ethyl acetate layers were dried over $MgSO_4$ and evaporated in vacuo to yield 49.74 of an amber oil. Trituration from 330 mL of diethyl ether (room temperature, overnight) oiled out phosphorous by-products. The ether solution was decanted away from the dark red oil into a separatory funnel, and the oil which was carried over by the decantation was drained off (1.56 g). Evaporation of the ether solution in vacuo yielded 43.08 g of [1S-[1α,2α(Z),3α,4α]]-6-[3-(hydroxymethyl)-7-oxabicyclo[2.2.1]hept-2-yl]-4-hexenoic acid in the form of a viscous yellow oil.

$^1$H NMR indicated a product: triphenylphosphine oxide: ether molar ratio of 23:1:1.8 (mass % 93:4.7:2.2). Yield exclusive of triphenylphosphine oxide/ether, 40.06 g (72.5%).

Acetyl chloride (5.20 mL, 0.073 mol) was added dropwise to 80 mL of methanol at room temperature under argon. The acetyl chloride/methanol solution was then added to a solution of 42.98 g (0.18 mol) of the preceding acid in 700 mL of methanol in one portion. Stirring was continued for 3 hours. Triethylamine was added (0.09 mol, 12.21 mL), methanol was removed in vacuo and the residue was partitioned between 300 mL of ethyl acetate and 150 mL of water. After separation of the layers, the aqueous layer was extracted with 150 mL of ethyl acetate, the combined ethyl acetate layers were washed with brine, dried over $Na_2SO_4$ and evaporated in vacuo to yield 43.06 g of a viscous tan oil. Flash chromatography on 1350 g of E. Merck Kieselgel 60 silica gel (240-400 mesh, 75/25 diethyl ether/hexanes, then diethyl ether after the desired product began eluting off the column) yielded 35.74 g title ester in the form of a viscous light yellow oil, free from triphenylphosphine oxide by NMR.

$^1$H NMR ($CDCl_3$, ref. TMS): δ 5.41-5.38, m (2H); 4.49, d, J=4.69Hz (1H); 4.22, d, J=4.69Hz (1H); 3.73-3.69, m (1H); 3.67, s, (3H); 3.60, m (1H); 2.37, br s (4H); 2.12-1.99, m (3H); 1.97-1.85, m (1H); 1.72, m (2H); 1.46, m (2H).

$^{13}$C NMR ($CDCl_3$, ref. 77.00): δ 173.50, 130.42, 128.63, 80.23, 79.22, 61.74, 51.49, 48.95, 46.45, 33.86, 29.69, 29.31, 25.94, 22.92.

D. N-(4-Cyclohexylbutyl) 1H-imidazol-4-carboxamide

A suspension of 0.30 g of Part a acid (2.68 mmol) in 3.0 mL of dimethylformamide (DMF) (room temperature, argon) was treated with 0.52 g (3.21 mmol) of 1,1′-carbonyldiimidazole. Stirring was continued overnight. Triethylamine (0.45 mL, 3.21 mmol) was added followed by Part B amine (0.62 g, 3.21 mmol). Stirring was continued for 6 hours. The DMF was removed in vacuo (vacuum pump, dry ice-cooled receiver flask). Ethyl acetate and 30 mL of 0.1M HCl were added. The ethyl acetate layer was washed with 0.1M HCl (3 times, 5 mL each), saturated aqueous $K_2CO_3$ solution (3 times, 5 mL each), brine, dried over $Na_2SO_4$ and concentrated to yield 0.67 g of a yellow solid. Trituration from 10 mL of hexanes (1 crop) yielded 0.63 g of title compound in the form of an off-white solid.

E. [1S-[1α,2α(Z),3α,4α]]-6-[3-[[[(4-Methylphenyl)sulfonyl]oxy]methyl]-7-oxabicyclo[2.2.1]hept-2-yl]-4-hexenoic acid, methyl ester

A solution of 4.00 g of Part C compound (15.73 mmol) in 200 mL of $CH_2Cl_2$ (room temperature, Ar) was treated with 19.08 mL of pyridine (235.95 mmol) then p-toluenesulfonyl chloride (5.97 g, 31.46 mmol). Stirring was continued for 3 days. TLC indicated the presence of unconsumed starting alcohol. An additional portion of p-toluenesulfonyl chloride was introduced (2.99 g, 15.73 mmol) and the reaction was continued overnight. Dichloromethane was removed under vacuum and replaced with 200 mL of diethyl ether. The cloudy ether solution was washed 5 times with 1M HCl (300 mL total), 1M NaOH solution (5 times, 250 mL total), brine, dried over $Na_2SO_4$ and evaporated in vacuo to yield an off-white sticky solid. Flash chromatography on 630 g of silica gel yielded 6.91 g of an off-white crystalline solid. The product was eluted with 60/40 hexanes/ethyl acetate after elution of p-toluenesulfonyl chloride with 85/15 hexanes/ethyl acetate. A minor contaminant was removed from the above product by a brief trituration with 25 mL of hexanes (0° C) followed by filtration and 2 rinses with 10 mL each of hexanes (0° C). Yield: 5.12 g of title compound as a white crystalline solid.

F. [1S-[1α,2α(Z),3α,4α]]-6-[3-[[4-[[(4-Cyclohexylbutyl)amino]carbonyl]-1H-imidazol-1-yl]methyl]-7-oxabicyclo-[2.2.1]hept-2-yl]-4-hexenoic acid, methyl ester

A solution of 0.31 g of Part D amide (1.23 mmol) in 2.0 mL of DMSO (room temperature, argon) was treated with 0.05 g of sodium hydride (1.23 mmol of a 60% mineral oil dispersion). Stirring was continued for 30 minutes. Part E compound was added (0.20 g, 0.49 mmol) and the reaction was run at 85° C (oil bath temperature) for 2 hours. DMSO was removed in vacuo (vacuum pump, dry ice-cooled receiver flask). Ethyl acetate (30 mL) and 1M HCl were added. The ethyl acetate layer was washed 3 times with 1M HCl, 3 times with saturated aqueous $K_2CO_3$ solution, brine, dried over $Na_2SO_4$ and concentrated to yield 0.39 g of a viscous yellow oil. Flash chromatography on 27 g of silica gel (99.5/0.5 $CH_2CL_2/CH_3OH$) yielded 0.16 g of title compound as a white crystalline solid. Also isolated was 0.16 g of unconsumed Part C compound.

## Example 10

[1S-[1α,2α(Z),3α,4α]]-6-[3-[[4-[[(4-Cyclohexylbutyl)amino]carbonyl]-1H-imidazol-1-yl]methyl]-7-oxabicyclo-[2.2.1]hept-2-yl]-4-hexenoic acid

A solution of 0.16 g of Example 9 ester (0.33 mmol) in 14.4 mL THF/2.9 mL $H_2O$ was treated with 3.29 mL of a 1M aqueous LiOH solution. The cloudy 2-phase reaction was stirred vigorously for 2 hours. The reaction mixture was cooled to 0° C and brought to pH 1.0 with 6M HCl. THF was removed in vacuo and replaced with chloroform. The aqueous layer was extracted 4 times with chloroform, the combined chloroform layers (total 25 mL) were washed with brine, dried over $Na_2SO_4$ and concentrated to yield 0.14 g of a white taffy (90%). Concentration of the aqueous layer to c.a. 50% of its original volume was followed by 4 additional extractions with 8 mL each of chloroform. The combined chloroform layers were dried over $Na_2SO_4$, combined with the above 0.14 g of product, and evaporated in vacuo to yield 0.15 g of a white taffy (96%). This material was combined with 0.10 g of material from a previous batch and flash

chromatographed on 18 g of silica gel. Elution with 99/1 ethyl acetate/PAW yielded a white solid which was dissolved in 4.0 mL of chloroform and filtered through a Gelman Acrodisc-CR disc (0.45 micron). Yield after chloroform removal: 0.14 g of a white solid; mp >50° C.

| Anal. calc'd for $C_{27}H_{41}N_3O_4 \cdot 0.46H_2O$: | |
|---|---|
| | C, 67.57; H, 8.80; N, 8.76 |
| Found: | C, 67.77; H, 8.80; N, 8.56. |

Examples of additional compounds in accordance with the present invention which may be prepared following the procedures outlined in the specification and working Examples include, but are not limited to the following.

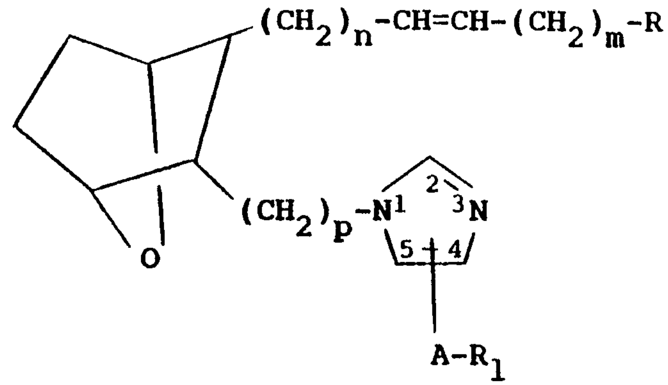

| Example No. | $(CH_2)_m$ m | $(CH_2)_n$ n | $(CH_2)_p$ p | A(position) | $R^1$ | R |
|---|---|---|---|---|---|---|
| 11 | 1 | 2 | 1 | CHOH-(2) | $-C_6H_{13}$ | $CO_2H$ |
| 12 | 2 | 2 | 2 | C=O(4) | $-(CH_2)_4-$ 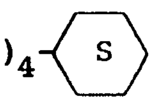 | $CO_2H$ |
| 13 | 3 | 1 | 1 | CHOH(5) | 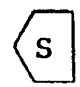 | $CONHSO_2CH_3$ |
| 14 | 1 | 2 | 1 | C=O(2) | $-C_2H_4-$ 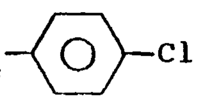 | 5-tetrazolyl |
| 15 | 2 | 3 | 1 | $C(O)NCH_3(2)$ | $C_6H_5$ | $CO_2H$ |
| 16 | 1 | 2 | 2 | -(4) | $-CH_2C_6H_5$ | 5-tetrazolyl |
| 17 | 1 | 2 | 3 | -(4) | $i-C_3H_7$ | $CONHSO_2C_6H_5$ |

| Example No. | $(CH_2)_m$ m | $(CH_2)_n$ n | $(CH_2)_p$ p | A(position) | $R^1$ | R |
|---|---|---|---|---|---|---|
| 18 | 1 | 3 | 1 | -(4) | $-CH_2-$ 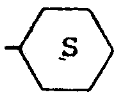 | $CONHSO_2CH_2C_6H_5$ |
| 19 | 1 | 2 | 1 | CHOH(5) | $-(CH_2)_3-$ 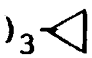 | $CO_2H$ |
| 20 | 2 | 2 | 3 | C=O(4) | 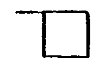 | $CO_2CH_3$ |
| 21 | 1 | 2 | 2 | C=O(4) | H | $CO_2Li$ |
| 22 | 1 | 3 | 1 | CHOH(5) | 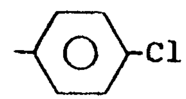 | $CO_2C_2H_5$ |
| 23 | 1 | 2 | 2 | -(2) | $(CH_2)_2C_6H_5$ | $CO_2H$ |
| 24 | 1 | 3 | 3 | - | H | 5-tetrazolyl |
| 25 | 1 | 2 | 1 | $C(O)NC_2H_5(5)$ | $n-C_5H_{11}$ | $CO_2H$ |
| 26 | 2 | 3 | 1 | C(O)NH(4) | 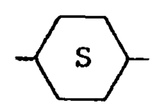 | 5-tetrazolyl |
| 27 | 1 | 2 | 1 | - | H | $CONHSO_2CH_3$ |

## Claims

1. A compound having the formula

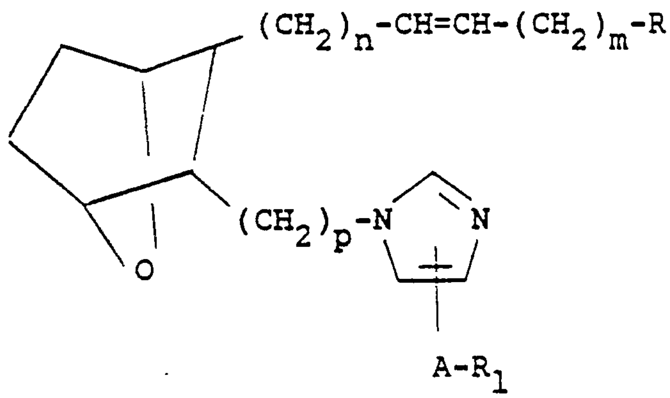

,

including all stereoisomers thereof, wherein
m is 1, 2 or 3;
n is 1, 2 or 3; p is 1, 2 or 3;
R is $CO_2H$, $CO_2$alkali metal, $CO_2$lower alkyl, $CONHSO_2R_2$ (wherein $R_2$ is lower alkyl or aryl) or 5-tetrazolyl;
A is CHOH, C=O,

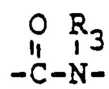

(wherein $R_3$ is H or lower alkyl), or a single bond;
$R_1$ is lower alkyl, aryl, cycloalkyl or H, $R_1$ can be H only when A is a single bond.

2. The compound as defined in Claim 1 having the formula

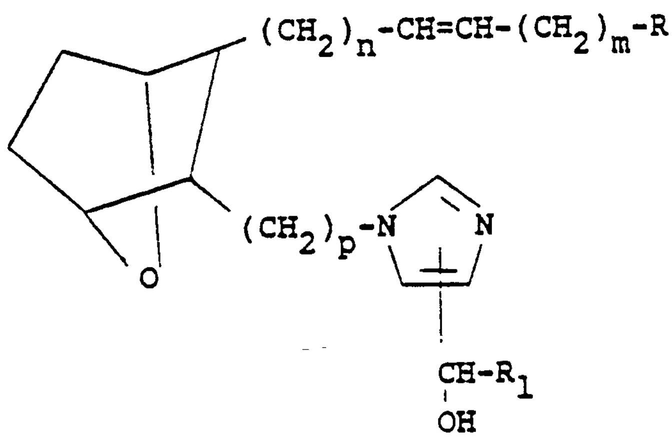

,

3. The compound as defined in Claim 2 where m is 2, n is 1 and p is 1.

4. The compound as defined in Claim 1 having the formula

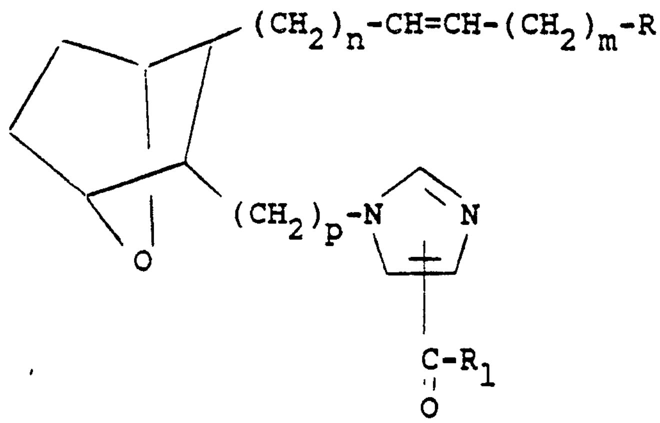

,

5. The compound as defined in Claim 4 wherein m is 2, n is 1 and p is 1.

6. The compound as defined in Claim 1 having the formula

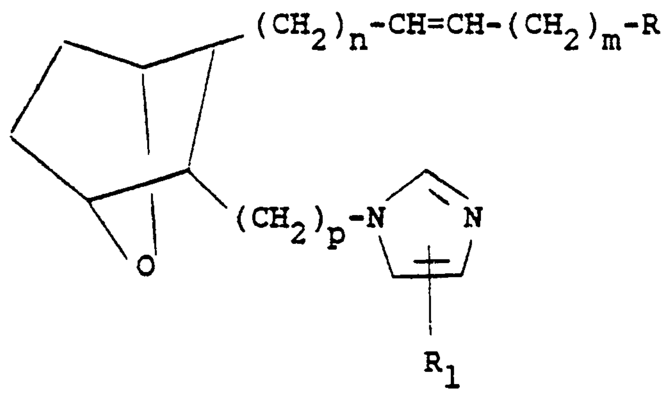

7. The compound as defined in Claim 6 wherein n is 1, m is 2 and p is 1.

8. The compound as defined in Claim 1 having the formula

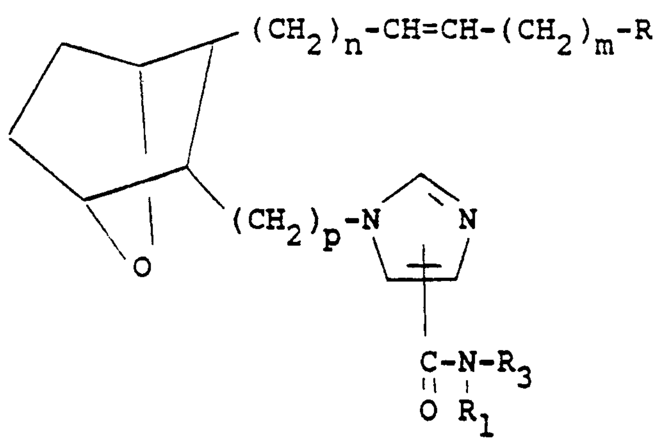

,

9. The compound as defined in Claim 8 wherein n is 1, m is 2 and p is 1.

10. The compound as defined in Claim 1 wherein R is $CO_2H$, $CONHSO_2R_2$ or 5-tetrazolyl.

11. The compound as defined in Claim 1 having the name [1S-[1α,2α(Z),3α,4α]]6-[3-[[4-(4-cyclohexyl-1-hydroxybutyl)-1H-imidazol-1-yl]methyl]-7-oxabicyclo[2.2.1]hept-2-yl]-4-hexenoic acid or its methyl ester.

12. The compound as defined in Claim 1 having the name [1S-[1α,2α(Z),3α,4α]]-6-[3-[[4-(3-cyclohexylpropyl)-1H-imidazol-1-yl]methyl]-7-oxabicyclo[2.2.1]hept-2-yl]-4-hexenoic acid or its methyl ester.

13. The compound as defined in Claim 1 having the name [1S-[1α,2α(Z),3α,4α]]-6-[3-[[4-(4-cyclohexyl-1-oxobutyl)-1H-imidazol-1-yl]methyl]-7-oxabicyclo[2.2.1]hept-2-yl]-4-hexenoic acid or its methyl ester.

14. The compound as defined in Claim 1 having the name [1S-[1α,2α(Z),3α,4α]]-6-[3-(1H- imidazol-1-ylmethyl)-7-oxabicyclo[2.2.1]hept-2-yl]-4-hexenoic acid or its methyl ester.

15. The compound as defined in Claim 1 having the name [1S-[1α,2α(Z),3α,4α]]-6-[3-[[4-[[(4-cyclohexylbutyl)amino]carbonyl]-1H-imidazol-1-yl]methyl-7-oxabicycylo[2.2.1]hept-2-yl]-4-hexenoic acid, or its methyl ester.

16. A composition for inhibiting platelet aggregation and bronchoconstriction comprising an effective amount of a compound as defined in any preceding claim, and a pharmaceutically acceptable carrier therefor.

17. Use of a compound as defined in any one of Claims 1-15 for the manufacture of a medicament for inhibiting platelet aggregation.

18. Use of a compound as defined in any one of Claims 1-15 for the manufacture of a medicament for inhibiting bronchoconstriction associated with asthma.

19. Use of a compound as defined in any one of Claims 1-15 for the manufacture of a medicament for improving post-ischemic myocardial dysfunction.

20. Use of a compound as defined in any one of Claims 1-15 for the manufacture of a medicament for treating toxemia during pregnancy.

21. Use of a compound as defined in any one of Claims 1-15 for the manufacture of a medicament for preventing or reducing venous thrombosis.

22. Use of a compound as defined in any one of Claims 1-15 for the manufacture of a medicament for preventing or reducing platelet loss during extracorporeal circulation.

23. Use of a compound as defined in any one of Claims 1-15 for the manufacture of a medicament for treating burn injuries and/or promoting wound healing in systemic or topical form.

24. Use of a compound as defined in any one of Claims 1-15 for the manufacture of a medicament for reducing post-ischemic myocardial injury, together with a thrombolytic agent within 8 hours of a myocardial infarction.

25. The use as defined in Claim 24 wherein said thrombolytic is t-PA, streptokinase, urokinase, prourokinase or anisoylated plasminogen-streptokinase activator complex.

26. A compound as defined in any one of Claims 1-15 for use as an active pharmaceutical substance.

27. A process for preparing a compound as defined in any one of Claims 1-15 comprising reacting a compound of formula H or H′ herein with a compound of the formula

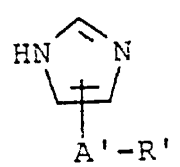

wherein A′ is:

(a) protected CHOH which is subsequently deprotected to form products wherein A is CHOH which can subsequently be oxidized to form products wherein A = C=O

(b) 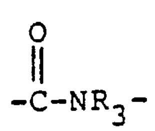 or

(c) single bond

and treating the above product wherein R is $CO_2$ alkyl to form a product where R is $CO_2H$

and further treating the above product with a sulfonamide of the formula P herein to form a product wherein R is $CONHSO_2R_2$

and if desired treating the above product wherein R is $CO_2H$ according to conventional methods to form a product where R is $CO_2$ alkali metal.

European Patent Office

# EUROPEAN SEARCH REPORT

Application number

EP 90305552.3

## DOCUMENTS CONSIDERED TO BE RELEVANT

| Category | Citation of document with indication, where appropriate, of relevant passages | Relevant to claim | CLASSIFICATION OF THE APPLICATION (Int Cl.5) |
|---|---|---|---|
| A | US - A - 4 687 865 (THOTTATHIL) * Abstract * | 1, 16-21, 24-27 | C 07 D 493/08<br>A 61 K 31/415 |
| A | EP - A2/A3 - 0 166 876 (E.R. SQUIBB & SONC) * Claim 1; page 17, lines 17 -28 * | 1, 16-21, 24-27 | |
| A | EP - A1 - 0 162 762 (E.R. SQUIBB & SONS) * Abstract; page 20, lines 6 -16 * | 1, 16-21, 24-27 | |

TECHNICAL FIELDS SEARCHED (Int Cl.5)

C 07 D 493/00

The present search report has been drawn up for all claims

| Place of search | Date of completion of the search | Examiner |
|---|---|---|
| VIENNA | 24-07-1990 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document